# EUROPEAN PATENT APPLICATION

(11) **EP 4 817 595 A1**
(43) Date of publication of application: **07.10.2026**
(21) Application number: 25305482.9
(22) Date of filing: 01.04.2025
(51) Int. Cl.: A61K 8/64, A61K 8/73, A61Q 5/06, A61Q 17/04, A61Q 19/00, A61Q 19/10

(54) **PERSONAL CARE COMPOSITION COMPRISING A NATURAL THICKENER**

(71) Applicant: SNF Group, 42160 Andrézieux-Bouthéon (FR)
(72) Inventor: VINCENT, Maxime, 42160 ANDREZIEUX-BOUTHEON (FR); SOUZY, Renaud, 42160 ANDREZIEUX-BOUTHEON (FR); BRAUN, Olivier, 42160 ANDREZIEUX-BOUTHEON (FR)
(74) Representative: Ipsilon

(57) **Abstract**

The invention relates to a personal care composition comprising a natural thickener, a process for preparing the natural thickener and said natural thickener.

## Description

### TECHNICAL FIELD OF THE INVENTION

The invention relates to a personal care composition comprising a natural thickener, a process for preparing the natural thickener and said natural thickener.

### TECHNICAL BACKGROUND

Thickeners are commonly used in home and personal care formulations to provide the desired viscosity and rheological properties to products like shampoos, lotions, dishwashing liquids and detergents, while also enhancing their stability.

They are mainly divided into two categories, natural thickeners and synthetic thickeners. Natural thickeners are generally derived from natural polymers such as xanthan gum or guar gum, which have the advantage of being biosourced and generally biodegradable. Whereas synthetic thickeners, such as polyacrylamides or polyethylene glycols, are derived from petroleum and are obtained through various chemical processes. They generally offer better applicative performances at the expense of their biodegradability. Another problem encountered for both categories of thickeners is maintaining their performance depending on the pH and high salt concentrations.

The Applicant has developed new natural thickeners in line with the principles of environmental consciousness, and the impact of industry and human activity on the planet. These new natural thickeners offer equivalent performances to synthetic polymers, while also having enhanced biodegradability. They also offer good performances as they keep their thickening properties at high salt concentrations and at over a wide range of acidic and basic pH values. The production method of these new natural thickeners is simple, safe and environmentally friendly with low energy consumption in particular by avoiding the neutralization step required for synthetic polymer which involves hazardous compounds and hard pH conditions.

The present invention is advantageously carried out using materials of biological origin, such as biomass, or recycled materials. The energy used to carry out the process according to the invention is advantageously derived from heat pumps, a waste heat network, a renewable source, for example wind power or photovoltaics, or from a fuel cell, a lithium battery or a nuclear power plant.

### DESCRIPTION OF THE INVENTION

The invention relates to a personal care composition comprising from 0.1 to 10 % by weight of a natural thickener, relative to the weight of the personal care composition, wherein the natural thickener has a particle size of between 1 µm and 1 000 µm and is prepared according to at least the following steps:
i) preparing a mixture M1 comprising at least one crosslinker and a solvent S1 able to solubilize, to disperse or to suspend the at least one crosslinker,
   wherein a weight ratio between the at least one crosslinker and the solvent S1 in the mixture M1 is between 0.1/99.9 and 30/70;
ii) forming the natural thickener by mixing the mixture M1 with at least one natural polymer in non-hydrated particle form, wherein a weight ratio between the at least one crosslinker and the at least one natural polymer in non-hydrated particle form is between 0.01/99.9 and 10/90, and wherein the mixing lasts between 1 minute and 150 minutes, at a temperature comprised between 20°C and 160°C.

The present invention also relates to a process for preparing a natural thickener comprising the following steps:
i) preparing a mixture M1 comprising at least one crosslinker and a solvent S1 able to solubilize, to disperse or to suspend the at least one crosslinker,
   wherein a weight ratio between the at least one crosslinker and solvent S1 in the mixture M1 is between 0.1/99.9 and 30/70;
ii) forming the natural thickener by mixing the mixture M1 with at least one natural polymer in non-hydrated particle form, wherein a weight ratio between the at least one crosslinker and the at least one natural polymer in non-hydrated particle form is between 0.01/99.9 and 10/90, and wherein the mixing lasts between 1 minute and 150 minutes, at a temperature comprised between 20°C and 160°C.

The present invention also relates to the natural thickener obtained by the above-mentioned process.

By "natural polymer" is meant any one of the followings: a polysaccharide as well as a modified polysaccharide, a protein as well as a modified protein, or a polypeptide as well as a modified polypeptide.

By "modified polysaccharide" is meant a polysaccharide that has undergone one or more treatments, which may be physical and/or chemical and/or enzymatic.

By "modified protein" is meant a protein that has undergone one or more treatments, which may be physical and/or chemical and/or enzymatic.

By "modified polypeptide" is meant a polypeptide that has undergone one or more treatments, which may be physical and/or chemical and/or enzymatic.

By "natural polymer in non-hydrated particle form" is meant a natural polymer in particle form that has not undergone any prior hydration treatment or any process that causes it to absorb a liquid and increase in volume, followed by a drying step.

By "natural thickener" is meant a crosslinked natural polymer which increases the viscosity of a liquid, preferably water.

As used herein, the expression "A and/or B" means "A, or B, or A and B".

The invention also includes all possible combinations of the various embodiments disclosed, whether they are preferred or exemplary embodiments when they are not mutually exclusive. Furthermore, where ranges of values are indicated, the end values are part of these ranges. The disclosure also includes all combinations between the end values of these ranges. For example, ranges "1-20, preferably 5-15", imply disclosure of the ranges "1-5", "1-15", "5-20" and "15-20".

The invention is based on the selection of specific natural polymers combined with a specific process for preparing a natural thickener.

All the particular and/or preferred embodiments described in the invention are combinable, provided they are not incompatible.

### Personal care composition

The invention relates to a personal care composition comprising from 0.1 to 10 % by weight of a natural thickener, relative to the weight of the personal care composition, wherein the natural thickener has a particle size of between 1 µm and 1000 µm and is prepared according to at least the following steps:
i) preparing a mixture M1 comprising at least one crosslinker and a solvent S1 able to solubilize, to disperse or to suspend the at least one crosslinker,
   wherein a weight ratio between the at least one crosslinker and the solvent S1 in the mixture M1 is between 0.1/99.9 and 30/70;
ii) forming the natural thickener by mixing the mixture M1 with at least one natural polymer in non-hydrated particle form, wherein a weight ratio between the at least one crosslinker and the at least one natural polymer in non-hydrated particle form is between 0.01/99.9 and 10/90, and wherein the mixing lasts between 1 minute and 150 minutes, at a temperature comprised between 20°C and 160°C.

The personal care composition can be any composition used in the personal care industry. It can be any of the followings: a shampoo, an aftershave, a sunscreen, a hand lotion, a liquid soap, a bar soap, a shaving cream, a conditioner, a hair dye, a perm, a relaxer, a hair bleach, a styling gel, a shower gel, a toothpaste, a face wash, a facial scrub, a makeup remover, a body lotion, a body butter, a bubble bath, a foundation, a mascara, an eyeshadow, an eyeliner, a foot cream, a baby shampoo, a baby lotion.

The personal care composition comprises from 0.1 and 10 % by weight of a natural thickener, relative to the weight of the composition, preferably from 0.2 and 5 % by weight, more preferably from 0.3 and 2 % by weight. The weight of the composition includes the weight of the natural thickener.

Depending on its function, the personal care composition can comprise one or more active ingredient.

By "active ingredient" is meant a compound which is conventionally used by the person skilled in the art. It provides specific properties to the composition, for instance care, softening, detangling, moisturizing....

Examples of active ingredients include any selected from the group consisting of: emollients, moisturizers, antioxidants, essential oils, humectants, revitalizing agents, UV filters, exfoliants, antimicrobial agents, antifungal agents, anti-dandruff agents, detangling agents, anti-static agents, cleansing agents, and exfoliating agents.

Examples of emollients include: cetyl alcohol, isopropyl myristate, squalane, caprylic/capric triglyceride, shea butter.

Examples of moisturizers include: hyaluronic acid, panthenol, glycerin, aloe vera extract, urea, chamomile extract.

Examples of antioxidants include: tocopherol (vitamin E), ascorbic acid (vitamin C), ferulic acid, butylated hydroxytoluene (BHT), butylated hydroxyanisole (BHA).

Examples of essential oils include: lavender oil, tea tree oil, eucalyptus oil, peppermint oil, lemon oil.

Examples of humectants include: glycerin, propylene glycol, sorbitol, sodium pyrrolidone carboxylic acid (PCA), butylene glycol.

Examples of revitalizing agents include: nicotinamide (niacinamide), ubiquinone (coenzyme Q10), hyaluronic acid, peptides.

Examples of UV filters include: butyl methoxydibenzoylmethane (avobenzone), 2-ethylhexyl 2-cyano-3,3-diphenylacrylate (octocrylene), zinc oxide, titanium dioxide, benzophenone-3 (oxybenzone).

Examples of antimicrobial agents include: phenoxyethanol, benzyl alcohol, chlorhexidine, triclosan, methylparaben.

Examples of antifungal agents include: climbazole, ketoconazole, zinc pyrithione, undecylenic acid, piroctone olamine.

Examples of anti-dandruff agents include: zinc pyrithione, selenium sulfide, ketoconazole, salicylic acid, piroctone olamine.

Examples of detangling agents include: silicones (dimethicone, cyclopentasiloxane), polyquaternium-7, cetrimonium chloride, hydrolyzed keratin, behentrimonium methosulfate.

Examples of conditioning agents include: behentrimonium chloride, cetrimonium bromide, polyquaternium-10, hydrolyzed silk, dimethicone.

Examples of antistatic agents include: cetrimonium chloride, behentrimonium chloride, quaternium-80, polyquaternium-10, stearamidopropyl dimethylamine.

Examples of cleansing agents include: sodium lauryl sulfate (SLS), sodium laureth sulfate (sodium lauryl ether sulfate), coco-glucoside, decyl glucoside, cocamidopropyl betaine, sodium cocoyl isethionate.

Examples of exfoliants include: salicylic acid, glycolic acid, lactic acid, jojoba beads, apricot kernel powder, polyethylene microbeads.

The person skilled in the art is able to select the active ingredients and the quantity thereof depending on the nature of the personal care composition.

Preferably, the personal care composition comprises between 0.1% and 30 % by weight of active ingredient, relative to the weight of the personal care composition.

Preferably, the personal care composition comprises one or more additives and/or one or more excipients.

Examples of additives and/or excipients include: emulsifiers, preservatives, colorants, chelating agents, opacifying agents, stabilizers, pH adjusters, rheology modifiers (excluding natural thickeners according to the invention), buffering agents, lubricants, chelators, pearlizing agents and fragrances.

Examples of emulsifiers include: lecithin, polysorbate 80, monoglyceride and diglyceride, glycerin, xanthan gum, sorbitan ester.

Examples of preservatives include: phenoxyethanol, benzyl alcohol, methylparaben, ethylhexylglycerin, sodium benzoate, potassium sorbate.

Examples of colorants include: titanium dioxide (CI 77891), brilliant blue FCF (CI 42090, blue 1), Allura Red AC (CI 16035, red 40), tartrazine (CI 19140, yellow 5), iron oxides (CI 77491/92/99).

Examples of chelating agents include: disodium EDTA, tetrasodium EDTA, sodium phytate, citric acid, gluconolactone.

Examples of opacifying agents include: glycol distearate, styrene/acrylates copolymer, titanium dioxide, mica, stearic acid.

Examples of stabilizers include: xanthan gum, carbomer, lecithin, polysorbates, magnesium aluminum silicate.

Examples of pH adjusters include: sodium hydroxide, citric acid, lactic acid, triethanolamine (TEA), sodium carbonate.

Examples of rheology modifiers include: carbomer, hydroxyethyl cellulose, xanthan gum, guar gum, acrylates/C₁₀₋₃₀ alkyl acrylate crosspolymer, sodium polyacrylate, polyacrylamide, sodium acrylate/acryloyl dimethyl taurate copolymer, ammonium acrylates copolymer.

Examples of buffering agents include: sodium bicarbonate, citric acid, potassium phosphate, trisodium citrate, lactic acid.

Examples of lubricants include: dimethicone, cyclopentasiloxane, isopropyl myristate, jojoba oil, squalane.

Examples of pearlescent agents include: glycol distearate, mica, titanium dioxide, guar hydroxypropyltrimonium chloride.

Examples of fragrances include: linalool, limonene, geraniol, citronellol, coumarin.

Preferably, the personal care composition comprises between 0.1 and 30 % by weight of one or more additives and/or one or more excipients, relative to the weight of the personal care composition.

The person skilled in the art is able to select the additive(s) and/or excipient(s) and the quantity thereof depending on the nature of the personal care composition.

The remainder of the composition is generally a continuous phase selected from: water, C₁-C₄ alcohol(s), oil(s), wax(es) and mixtures thereof. For anhydrous personal care compositions such as bar soap, eyeshadow, eyeliner, and some body butters and foundations, only oil(s) and/or wax(es) are used.

Advantageously, the personal care composition comprises, relative to the weight of the personal care composition:
- between 0.1% and 10% of one or more natural thickener(s);
- between 0.1% and 30% of one or more active ingredient(s);
- between 0.1% and 30% of one or more additive(s) and/or excipient(s);
- between 30% and 99.7% of a continuous phase selected from water, C₁-C₄ alcohol(s), oil(s), wax(es) and mixtures thereof.

### Preparing the natural thickener comprised in the personal care composition

### Step i)

Step i) corresponds to the preparation of a mixture M1 comprising at least one crosslinker and a solvent S1 able to solubilize, to disperse or to suspend the crosslinker.

The crosslinker is advantageously selected from compounds having at least two reactive functions able to react with a natural polymer. The crosslinker is preferably selected from the group consisting of: polyepoxys, polycarboxylic acids, anhydride derivatives, polycarbamides, polyamides, polyamines, polyaldehydes, polyols, polyvalent inorganic salts, and mixtures thereof.

Preferably, polyepoxys are selected from diepoxys, triepoxys, tetraepoxys and pentaepoxys. Examples of diepoxys include diepoxypolyethylene glycol preferably having a molecular weight comprised between 200 and 6000 g/mol, more preferably between 500 and 2000 g/mol. Examples of triepoxys include polyglycidyl ether ethoxylate trimethylolpropane and triglicidylamine. Examples of tetraepoxys include sorbitol polyglycidyl ether and polyglycerol polyglycidyl ether. Examples of pentaepoxys include polyglycerol polyglycidyl ether, pentaglycidyl ether of xylitol, pentaglycidyl ether of sorbitol and pentaglycidyl ether of pentaerythritol.

Polycarboxylic acids are advantageously selected from: triacids and tetraacids. Examples of triacids include citric acid, nitrilotriacetic acid and tricarballylic acid. Examples of tetraacids include ethylenediaminetetraacetic acid and 1,2,3,4-butanetetracarboxylic acid.

Anhydride derivatives are advantageously selected from the group consisting of: adipic anhydride, itaconic anhydride and maleic anhydride.

Polyamides can be obtained by polycondensation of citric acid and urea.

Polyamines are advantageously selected from the group consisting of: diamines, triamines, and tetraamines. Examples of diamines include ethylenediamine, piperazine and tetramethylenediamine. Examples of triamines include diethylenetriamine, ethylenetriamine and trifunctional polyetheramine. Example of tetraamines include triethylenetetramine.

Dialdehydes are advantageously selected from the group consisting of: glyoxal, glutaraldehyde, 2,5-furandicarboxaldehyde, adipaldehyde, succinaldehyde, polysaccharide multialdehyde and 2,2-dimethoxyethanal.

Polyols are advantageously selected from: glycerin and sorbitol.

Inorganic salts are advantageously selected from the group consisting of: aluminum sulfate octadecahydrate, ammonium zirconium carbonate, calcium chloride, zinc chloride, iron chloride and magnesium chloride.

Preferably, the crosslinker is selected from the group consisting of: triacids, tetraacids, tetraamines, polyols, inorganic salts, diepoxys, triepoxys, tetraepoxys, pentaepoxys. Preferably, the crosslinker is a pentaepoxy compound.

The quantity of crosslinker is advantageously between 10 ppm and 100,000 ppm based on the weight of natural polymer, preferably between 100 ppm and 10,000 ppm, more preferably between 500 ppm and 5,000 ppm, and even more preferably between 1,000 ppm and 3,000 ppm.

The solvent S1 able to solubilize, to disperse or to suspend the crosslinker is advantageously selected from the group consisting of: C₁-C₄ alcohols; C₃-C₄ ketones; mineral oils; vegetable oils; C₁-C₈ linear ethers; C₁-C₈ cyclic ethers; C₁-C₈ branched ethers; and mixtures thereof.

Preferably, the solvent S1 is C₁-C₄ alcohol selected from the group consisting of: methanol, ethanol, 1-propanol, isopropanol, 2-methyl-1-propanol, tert-butanol, 1-butanol, 2-butanol, and mixtures thereof.

Ketones having between 3 and 4 carbon atoms are advantageously selected from the group consisting of: dimethyl ketone, diethyl ketone, 2-pentanone, butanone, tetrahydropyran, tetrahydrofuran, 2-methyltetrahydrofuran, 1,3-dioxane, 1,4-dioxane and mixtures thereof.

Mineral oils are advantageously selected from the group consisting of: Isopar^{™} E, Isopar^{™} J, Isopar^{™} L, Isopar^{™} M, vegetable oils, and mixtures thereof. Vegetable oils are advantageously selected from the group consisting of: squalene, an ester-type or triglyceride-type oil, like coco caprylate/caprate, octyldodecyl myristate, ethoxylated vegetable oils, jojoba oil, macadamia oil, and mixtures thereof.

Linear, cyclic or branched ethers having between 1 and 8 carbon atoms are advantageously selected from tetrahydrofuran and petroleum ether.

Preferably, the solvent S1 is a C₁-C₄ alcohol selected from the group consisting of: methanol; ethanol; 1-propanol; isopropanol; 2-methyl-1-propanol; tert-butanol; 1-butanol; 2-butanol; and mixtures thereof. Preferably the C₁-C₄ alcohol is isopropanol.

The solvent S1 can comprise traces of water. Preferably, the solvent S1 comprises less than 15 000 ppm by weight of water, more preferably less than 10 000 ppm by weight, more preferably less than 5 000 ppm by weight and even more preferably less than 1 000 ppm by weight.

In a preferred embodiment, there is no co-solvent mixed to the solvent S1.

The mixture M1 comprises a weight ratio between the at least one crosslinker and the solvent S1 comprised between 0.1/99.9 and 30/70 (between 0.001 and 0.429), preferably between 1/99 and 15/85 (between 0.010 and 0.176), and more preferably between 1.5/98.5 and 10/90 (between 015 and 0.111).

### Step ii)

Step ii) comprises the formation of the natural thickener by mixing the mixture M1 with at least one natural polymer in non-hydrated particle form, for between 1 minute and 150 minutes, at a temperature comprised between 20°C and 160°C. The weight ratio between the at least one crosslinker and the least one natural polymer in non-hydrated particle form is between 0.01/99.99 and 10/90 (between 0.0001 and 0.1111).

The "particle size", refers to the size of particles measured by conventional techniques, for instance with a laser measuring device such as Zetasizer^{®} Nano by Malvern^{®}. Regardless of the shape of a particle, the particle size corresponds to the diameter of the sphere scattering in the same way as the particle. In the present invention, the "particle size" refers to the particle sizer distribution based on number distribution. For example, D50 is the mean particle size wherein 50% of the distribution is smaller than the D50 value, and 50% is bigger. Similarly, 90% percent of the population is smaller than the D90 value, and 10% is bigger. D10 is the particle size wherein 10% of the population is smaller than the D10 value, and 90% is bigger.

Optionally, the natural polymer in non-hydrated particle form is grinded and sieved to a particle size of between 1 µm and 1000 µm before step ii), i.e. before being mixed with mixture M1. The natural polymer in non-hydrated particle form has a particle size of between preferably between 2 µm and 500 µm, more preferably between 5 µm and 150 µm.

Optionally, the natural thickener is grinded and sieved to a particle size of between 1 µm and 1 000 µm in step ii).

Preferably, the natural thickener has a particle size of between 1 µm and 1 000 µm, preferably between 20 µm and 1 000 µm, more preferably between 40 µm and 200 µm, even more preferably between 100 µm and 150 µm. Preferably, this particle size is D90.

Advantageously, the natural thickener has a D50, comprised between 10 µm and 500 µm, preferably between 30 µm and 100 µm, more preferably between 50 µm and 70 µm.

Advantageously, the natural thickener has a D10, comprised between 1 µm and 50 µm, preferably between 10 µm and 30 µm, more preferably between 20 µm and 30 µm.

Preferably, the sieving of the natural thickener results in a uniform particle size distribution, which may be advantageous depending on the nature of the personal care composition. The sieving can be carried out using any means known by the person skilled in the art, for example any one of the following techniques: a vibratory sifter, a rotary sifter, an ultrasonic sieve, an inline sifter, a gyratory screener or an air classifier.

The grinding can be carried out by any means known by the person skilled in the art. For example, it can be done using any one of the following techniques: a ball mill, a blade grinder, a cryogenic grinder, a jet mill, a hammer mill, a pin mill, a roller mill, an attrition mill, an ultrafine grinder, a vibratory mill, a planetary ball mill, a disc mill, a universal mill, an impact mill.

The natural polymer particles can be in the form of powder, granules, agglomerates or pellets. Preferably, the natural polymer particles are in powder form.

The natural polymer has advantageously a particle size comprised between 1 µm and 1 000 µm, preferably between 20 µm and 1 000 µm, more preferably between 40 µm and 200 µm, even more preferably between 100 µm and 150 µm. Preferably, this particle size is D90.

Advantageously, the natural polymer has a D50 comprised between 10 µm and 500 µm, preferably between 30 µm and 100 µm, more preferably between 50 µm and 70 µm.

Advantageously, the natural polymer has a D10 comprised between 1 µm and 50 µm, preferably between 10 µm and 30 µm, more preferably between 20 µm and 30 µm.

In a preferred embodiment, the natural polymer in non-hydrated particle form is not crosslinked before the mixing with mixture M1.

By "not crosslinked" is meant a natural polymer in which the molecular chains are not interconnected by additional covalent bonds. **In** that case, the natural polymer is substantially free of interconnections between chains. The natural polymer can be linear or branched.

**In** a preferred embodiment, the natural polymer is a mixture of different natural polymers.

The natural polymer is preferably selected from the group consisting of: a polysaccharide, a modified polysaccharide, a protein, a modified protein, a polypeptide or a modified polypeptide and mixture thereof. Preferably, the natural polymer in non-hydrated particle form is a polysaccharide.

The polysaccharide is preferably extracted from plants, animals or produced by microorganisms such as bacteria, fungi, prokaryotes and eukaryotes. For example, xanthan gum is preferably produced by Xanthomonas campestris, gellan by Sphingomonas paucimobllis, while xyloglucan is preferably extracted from tamarind seeds.

The polysaccharide is advantageously selected from the group consisting of: starch; dextrin; maltodextrin; tamarind gum (preferably consisting of xyloglucan); guar gum; konjac gum; tara gum; gelatin; locust bean gum (preferably consisting of galactomannan); carboxymethyl cellulose; pectin; polymers extracted such as, fenugreek, aloe vera, chia seeds, linseeds, psyllium seeds, quince seeds; tara gum; xanthan gum; gellan gum; welan gum, rhamsan gum; dextran; curdlan; pullulan; scleroglucan; schizophyllan; chitin; hydroxyalkyl cellulose; arabin (preferably obtained from sugar beet); arabinoxylane; galactans (preferably obtained from lupins and/or potatoes); pectic galactans (preferably obtained from potatoes); galactomannan (preferably obtained from carob);glucomannan; lichenan (preferably obtained from Iceland moss); mannan (preferably obtained from ivory nut); pachyman; rhamnogalacturonan; acacia gum; agar; alginates; carrageenans; chitosan; clavans; hyaluronic acid; heparin; inulin; cellodextrins; cellulose; cellulose derivatives and mixtures thereof.

In a particular embodiment, the polysaccharide undergoes chemical grafting reactions with various synthetic substituents, for example: carboxyalkyl starch, like carboxymethyl starch; carboxyalkyl cellulose, like carboxymethyl cellulose; carboxyalkyl guar, like carboxymethyl guar; hydroxypropyl methylcellulose; carboxyalkyl hydroxyalkyl cellulose, like carboxymethyl hydroxypropyl cellulose; carboxyalkyl hydroxyalkyl guars, like carboxymethyl hydroxypropyl guar. These types of reaction are familiar to those skilled in the art, and remain classic chemical reactions applied to natural polymers.

In a preferred embodiment, the polysaccharide is selected from the group consisting of: xanthan gum, guar gum, pectin, carrageenans, chitosan, cellulose, konjac gum, gelatin, alginates and mixtures thereof. More preferably, it is xanthan gum, konjac gum or a mixture thereof.

As already mentioned, the natural polymer can be a protein or a modified protein. The protein preferably originates from an animal, a vegetable, a bacterium, or is derived from a fungus, an algae or a yeast.

Animal proteins include milk proteins such as β-lactoglobulin, casein and whey; serum proteins such as horse serum; fibrous dermal proteins such as collagen, elastin and silk proteins.

Vegetable proteins include proteins extracted from corn, wheat, barley, oats, soya or peas, for instance from glutelin, prolamin, zein or gluten. Advantageously, vegetable proteins are selected from the group consisting of: soy, wheat, oat and pea proteins. Proteins can also be obtained from seeds (e.g. soy), cotton, peanut, sunflower, rapeseed, coconut, linseed, sesame, peas, beans or lentils.

Bacterial proteins include Pseudomonas, Lactobacillus and E.Coli. Fungi proteins include Penicillium. Algae proteins include blue-green algae; green algae such as Chlorella and Spirulina.

In a preferred embodiment, the protein is selected from the group consisting of: casein, serum proteins and wheat proteins.

The polypeptide preferably originates from an animal, a vegetable, a bacterium, or is derived from a fungus, an algae or a yeast. The polypeptide is advantageously selected from: polyglutamate, modified polyglutamate, polyaspartate, Preferably, the polypeptide is a polyglutamate.

The mixing of the mixture M1 and the at least one natural polymer in non-hydrated particle form can be done by any means known by the person skilled in the art, preferably by means of a stirring blade, spinning knives, rotating drums, an overhead anchor, a centrifugal pump, a static mixer, or a rotor/stator combination. Preferably, the mixing is done with an overhead anchor or a stirring blade.

The mixing is advantageously carried out at a rotation speed of between 10 and 10,000 rpm (rpm = rotation per minute), preferably between 100 and 5,000 rpm, more preferably between 200 and 1,000 rpm.

The mixing advantageously lasts between 1 minute and 3,000 minutes, preferably between 5 minutes and 2,000 minutes, more preferably between 10 minutes and 240 minutes and even more preferably between 20 minutes and 120 minutes.

The mixing time and the rotation speed are adjusted depending (1) on the agitator design and mixing vessel volume and (2) on the quantity of natural polymer. The person skilled in the art is able to ensure homogeneous mixing of the mixture M1 with the natural polymer by adjusting these parameters.

In a preferred embodiment, mixture M1 is sprayed on the natural polymer in non-hydrated particle form during the mixing step ii).

The spraying of mixture M1 can be done by any means known by the person skilled in the art, for example by dropwise addition and/or by spraying using a nozzle.

The mixing is carried out at a temperature of between 20°C and 160°C, preferably between 40°C and 150°C, more preferably between 50°C and 140°C, and even more preferably between 60°C and 120°C.

The mixing is advantageously carried out at atmospheric pressure.

Preferably, the natural thickener resulting from step ii) is dried by any means known by the person skilled in the art. This optional drying step allows removing residual traces of solvent.

Drying is advantageously carried out at a temperature of between 20°C and 160°C, preferably between 40°C and 150°C, more preferably between 50°C and 140°C, and even more preferably between 60°C and 120°C.

In step ii), the weight ratio between the at least one crosslinker and the least one natural polymer in non-hydrated particle form is between 0.01/99.99 and 10/90 (approximately 0.0001 to 0.1), advantageously between 0.05/99.95 and 7/93 (approximately 0.0005 to 0.0753), preferably between 0.1/99.9 and 5/95 (approximately 0.0010 to 0.0523).

### Process for preparing a natural thickener

The present invention also relates to a process for preparing a natural thickener comprising the following steps:
i) preparing a mixture M1 comprising at least one crosslinker and a solvent S1 able to solubilize, to disperse or to suspend the at least one crosslinker,
   wherein a weight ratio between the at least one crosslinker and solvent S1 in the mixture M1 is between 0.1/99.9 and 30/70;
ii) forming the natural thickener by mixing the mixture M1 with at least one natural polymer in non-hydrated particle form, wherein a weight ratio between the at least one crosslinker and the at least one natural polymer in non-hydrated particle form is between 0.01/99.9 and 10/90, and wherein the mixing lasts between 1 minute and 150 minutes, at a temperature comprised between 20°C and 160°C.

Preferably, the natural thickener resulting from step ii) has a particle size of between 1 µm and 1 000 µm, preferably between 20 µm and 1 000 µm, more preferably between 40 µm and 200 µm, even more preferably between 100 µm and 150 µm. Preferably, this particle size is D90.

Advantageously, the natural thickener has a D50, comprised between 10 µm and 500 µm, preferably between 30 µm and 100 µm, more preferably between 50 µm and 70 µm.

Advantageously, the natural thickener has a D10, comprised between 1 µm and 50 µm, preferably between 10 µm and 30 µm, more preferably between 20 µm and 30 µm.

The different embodiments previously described regarding the preparation of the natural thickener in the personal care composition also apply to the process of the invention.

Preferably, the process according to the invention is carried out using energy derived from a heat pump, a waste heat network, a renewable source, for example wind power or photovoltaics, from a fuel cell, a lithium battery, or a nuclear power plant.

The present invention also relates to the natural thickener prepared according to the process of the invention.

### EXAMPLES

### Starting raw materials

Xanthan gums used in the examples are the following:
- Xanthan gum 1: commercial name KELZAN^{®} AP-AS, provided by CP Kelco^{®};
- Xanthan gum 2: commercial name Xanthan Gum FF-PC, provided by Jungbunzlauer^{®};
- Xanthan gum 3: commercial name Xanthan Gum FEDCS-PC, provided by Jungbunzlauer^{®}. Guar gum used in the examples was provided by India Glycols Limited under the commercial name IGGUAR FG 5000.

Starch used in the examples was provided was provided by Roquette Frères under the commercial name Stabilys^{®} A020.

Poly(glutamic acid) (PGA) (700 000 g/mol) used in the examples was provided by Shandong Freda Biotechnology Co., Ltd.

Konjac gum was provided by Hubei Yizhi Konjac Biotechnology Co., Ltd under the commercial name ES-H03.

Crosslinkers used in the examples are:
- X1: Polyethylene glycol 500 diglycidyl ether was purchased from Sigma Aldrich^{®};
- X2: Polyglycidyl ether ethoxylate trimethylolpropane was purchased from Ipox Chemicals^{®};
- X3: Sorbitol polyglycidyl ether was purchased from Nagase^{®};
- X4: Polyglycerol polyglycidyl ether was purchased from Nagase^{®};
- X5: Polyethylene glycol/polypropylene glycol copolymer-based diamine was provided by Huntsman^{®}.

Synthetic thickeners used in the examples are:
- Lubrizol^{®} Carbopol^{®} 940;
- Tinci Materials Technology Co., TC-Carbomer^{®} 640;
- Evonik^{®} Industries Rheocare^{®} C Plus;
- Corel Pharma Chem Acritamer 940;
- Guangzhou DX Chemical Co., Ltd. Lingel 940;
- Ashland^{®} Global Aquastyle^{®} 940.

### Analysis

Bulk viscosity was measured using a Brookfield rheometer RVT. The choice of spindle and speed depends on the viscosity range:
- For formulations with viscosities between 8 000 and 15 000 cPs: Spindle #4/Speed: 20 rpm (rotation per minute).
- For formulations with viscosities under 10 000 cPs: Spindle #3/Speed: 60 rpm.
- For formulations with viscosities over 15 000 cPs: Spindle #5/Speed: 20 rpm.

Clarity was measured by centrifuging the samples at 8 000 rpm for 5 minutes in quartz cells. The prepared quartz cells were then placed in the DR 3900 Spectrophotometer from Hach Lange^{®}. A unique wavelength of 420 nm was used to measure the percentage of transmittance based on distilled water as reference for 100% transmittance.

The biodegradability of the polymers was assessed according to OECD 301F procedure. The results are presented as the oxygen consumption relative to the theoretical oxygen demand (ThOD).

A polymer is considered as inherently biodegradable if the biodegradability reaches less than 60% in 28 days according to OECD 301F.

A polymer is considered as readily biodegradable if the biodegradability reaches at least 60% in 28 days according to OECD 301F.

A polymer is considered as ultimately biodegradable if the biodegradability reaches at least 60% in 90 days according to OECD 301F.

Particle size was analyzed by laser diffraction. The device used is the Mastersizer^{®} MS3000 marketed by the Malvern company. This device is used to measure particle sizes in the range of 0.5 µm to 2000 µm. It is also used to measure particle size distribution. This device has an organic liquid channel for the analysis of aqueous polymer particles emulsified in oil. The device is equipped with three main elements that enable the system to consistently and reliably provide accurate particle size data:
- Optical bench: equipped with a laser beam (1 single optical measurement path);
- Sample dispersion units and Mastersizer^{®} 3000 software: it controls the system during measurement and analyzes the diffusion data.

### Example 1: Preparation of a natural thickener

89.74 g of xanthan gum 1 was introduced into a 1 Liter glass reactor equipped with a mechanical stirrer. The powder was stirred at 200 rpm using a magnetic stirrer.

### Preparation of M1:

We introduced in a beaker 0.259 g of polyglycerol polyglycidyl ether (X4) and 9.999 g of isopropanol. The resulting M1 mixture was stirred at 300 rpm at room temperature for 10 minutes using a three-blade stirrer.

### Formation of the natural thickener:

The mixture M1 was added at room temperature to the glass reactor comprising the xanthan gum 1 for 15 minutes. Then, the reaction medium was stirred during an additional 5 minutes to obtain the natural thickener N4.

The natural thickener N4 was heated at 100°C for 60 minutes and sieved to obtain the desired particle size.

Other natural thickeners N1, N2, N3, and N5 to N32 were prepared according and the previously described method by modifying the raw materials, solvent, weight ratio between the crosslinker and the solvent in M1 and weight ratio between the crosslinker and the natural polymer. The temperature and duration of drying were adapted to obtain a final dry natural thickener.

The compositions of the different natural thickeners N1 to N32 are summarized in Table 1.

**Table 1: Composition and nature of natural thickeners N1 to N32 (Inv: Invention, CE: Counterexample)**

| **Natural thickener** | **Natural polymer** | | | **M1** | | | **Weight ratio crosslinker /natural polymer** | **Particle size (µm)** |
|---|---|---|---|---|---|---|---|---|
| | **Raw material 1** | **Raw material 2** | **Weig ht ratio (Raw 1/ Raw 2)** | **Solvent S1** | **Crosslinker** | **Weight ratio crosslinker/solv ent S1** | | |
| **N1(CE)** | Xanthan gum 1 | - | 100/0 | - | - | - | - | 70 |
| **N2(CE)** | Xanthan gum 1 | - | 100/0 | Isopropanol | - | - | - | 70 |
| **N3(Inv)** | Xanthan gum 1 | - | 100/0 | Isopropanol | X4 | 10/90 | 0.0111 | 70 |
| **N4(Inv)** | Xanthan gum 1 | - | 100/0 | Isopropanol | X4 | 2,5/97,5 | 0.0028 | 70 |
| **N5(Inv)** | Xanthan gum 1 | - | 100/0 | Isopropanol | X4 | 2,5/97,5 | 0.0044 | 70 |
| **N6(Inv)** | Xanthan gum 1 | - | 100/0 | Isopropanol | X3 | 8/92 | 0,0412 | 70 |
| **N7(Inv)** | Xanthan gum 1 | - | 100/0 | Isopropanol | X3 | 2,5/97,5 | 0.0044 | 70 |
| **N8(Inv)** | Xanthan gum 1 | - | 100/0 | Isopropanol | X5 | 25/75 | 0,0247 | 70 |
| **N9(Inv)** | Xanthan gum 1 | - | 100/0 | Isopropanol | X1 | 10/90 | 0,0124 | 70 |
| **N10(Inv)** | Xanthan gum 1 | - | 100/0 | Ethanol | X4 | 3/97 | 0.0026 | 70 |
| **N11(Inv)** | Xanthan gum 1 | - | 100/0 | Acetone | X4 | 2/98 | 0.0022 | 70 |
| **N12(Inv)** | Xanthan gum 1 | - | 100/0 | Isopar E | X3 | 10/90 | 0,0333 | 70 |
| **N13(Inv)** | Xanthan gum 2 | - | 100/0 | Isopropanol | X4 | 2/98 | 0.0022 | 70 |
| **N14(Inv)** | Xanthan gum 2 | - | 100/0 | Tert-butanol | X4 | 5/95 | 0.0026 | 70 |
| **N15(CE)** | Xanthan gum 3 | - | 100/0 | - | - | - | - | 1000 |
| **N16(Inv)** | Xanthan gum 3 | - | 100/0 | Isopropanol | X4 | 3/97 | 0.0034 | 1 000 |
| **N17(Inv)** | Xanthan gum 3 | - | 100/0 | Isopropanol | X4 | 5/95 | 0.0050 | 1 000 |
| **N18(Inv)** | Xanthan gum 3 | | 100/0 | Isopropanol | X4 | 5/95 | 0.0050 | 70 |
| **N19(Inv)** | Xanthan gum 3 | | 100/0 | Isopropanol | X2 | 5/95 | 0.0050 | 70 |
| **N20(Inv)** | Xanthan gum 1 | Konjac gum | 50/50 | Isopropanol | X4 | 3/97 | 0.0034 | 70 |
| **N21(Inv)** | Xanthan gum 2 | Konjac gum | 50/50 | Isopropanol | X4 | 3/97 | 0.0034 | 70 |
| **N22(Inv)** | Xanthan gum 3 | Konjac gum | 50/50 | Isopropanol | X4 | 3/97 | 0.0034 | 1 000 |
| **N23(Inv)** | Konjac gum | Guar Gum | 50/50 | Isopropanol | X4 | 3/97 | 0.0034 | 1 000 |
| **N24(Inv)** | Konjac Gum | Guar Gum | 25/75 | Isopropanol | X4 | 5/95 | 0.0050 | 1 000 |
| **N25(Inv)** | Starch | - | 100/0 | Isopropanol | X3 | 9/91 | 0.0089 | 1 000 |
| **N26(Inv)** | Guar gum | - | 100/0 | Isopropanol | X3 | 3/97 | 0.0016 | 1 000 |

### Example 2: Biodegradability measurements

The biodegradability according to OECD 301F of natural thickeners N1, N7, N15, N17, N20, N22, N23, and N27 according to OECD 301F was determined and compared to the biodegradability of synthetic thickeners Carbopol^{®} 940, TC-Carbomer^{®} 640 and Rheocare^{®} C Plus. The results are resumed in Table 2.

**Table 2: Biodegradability of different thickeners**

| **Thickener** | **Biodegradability test at 30 days (%)** | **Biodegradability test at 60 days (%)** |
|---|---|---|
| **N1 (CE)** | 63 | 73 |
| **N7 (Inv)** | 41 | 45 |
| **N15 (CE)** | 70 | 80 |
| **N17 (Inv)** | 65 | 74 |
| **N20 (Inv)** | 59 | 61 |
| **N22 (Inv)** | 68 | 80 |
| **N23 (Inv)** | 48 | 52 |
| **N27 (Inv)** | 55 | 62 |
| **Carbopol^{®} 940 (CE)** | <10 | <20 |
| **TC-Carbomer^{®} 640 (CE)** | <10 | <20 |
| **Rheocare^{®} C Plus (CE)** | <10 | <20 |

### Example 3: Lightweight hydrating gel formulations

Different lightweight hydrating gel formulations F1-F9 were produced by replacing the nature of the thickener. The nature of the different thickeners comprised in F1 to F9 is summarized in Table 3.

### Ingredients (by weight %):

- Deionized Water: *q.s* (Quantum Satis)
- Glycerin: 3.0
- Thickener: 0.4
- Sodium chloride: 0.5
- Propylene glycol: 4.9
- Phenoxyethanol: 0.7
- Fragrance: 0.5
- Aloe Vera Extract: 6.0.

### Lightweight hydrating gel formulation protocol:

The thickener was dispersed in deionized water comprising sodium chloride, by using a low-speed mixer at 200 rpm, until a lumps-free solution was obtained. Glycerin was then added, and the solution was mixed for 20 minutes. For formulation F2 comprising the synthetic thickener, triethanolamine was slowly added to the mixture for 30 minutes, to adjust the pH to a range of 5.5 to 6.5.

The mixture was then stirred at 400 rpm until it became clear. Propylene glycol, phenoxyethanol, fragrance, and aloe vera extract were finally introduced. The mixture was then mixed until it became smooth and homogeneous.

The bulk viscosity of the different formulations F1 to F9 was measured and the results are outlined in Table 3.

**Table 3: Bulk viscosity of lightweight hydrating gel formulations F1 to F9**

| **Formulation** | **Thickener** | **Bulk viscosity (cps)** |
|---|---|---|
| **F1 (CE)** | **N15 (CE)** | 2000 |
| **F2 (CE)** | **Carbopol^{®} 940 (CE)** | 7 500 |
| **F3 (CE)** | **N31 (CE)** | 2 400 |
| **F4 (Inv)** | **N32 (Inv)** | 4 400 |
| **F5 (Inv)** | **N17 (Inv)** | 4 500 |
| **F6 (Inv)** | **N18 (Inv)** | 4 900 |
| **F7 (CE)** | **N1 (CE)** | 1 800 |
| **F8 (CE)** | **N2 (CE)** | 1 850 |
| **F9 (Inv)** | **N22 (Inv)** | 8 500 |

### Example 4: Body wash formulations

Different body wash formulations F10-F16 were produced by replacing the nature of the thickener. The nature of the different thickeners comprised in formulations F10 to F16 is summarized in Table 4.

### Ingredients (by weight %):

- Deionized water: *q.s*
- Thickener: 0.1
- Sodium laureth sulfate: 20
- Glycerin: 3
- Phenoxyethanol: 0.5
- Fragrance: 0.3.

### Body wash formulation protocol:

The thickener was slowly incorporated in deionized water while stirring at 200 rpm, until a lumps-free solution is obtained. For formulation F11 and F12 comprising synthetic thickeners, triethanolamine was slowly added to the mixture for 30 minutes to adjust the pH to a range of 5.5 to 6.5.

Sodium laureth sulfate and glycerin were added to the solution which was mixed for 20 minutes. The pH was adjusted, if needed, by using triethanolamine or citric acid to keep it in the range of 5.5 to 6.5. Phenoxyethanol and fragrance were finally introduced. The mixture was then mixed at 400 rpm until it formed a smooth and homogeneous formulation.

The bulk viscosity of the different formulations F10 to F16 was measured and the results are outlined in Table 4.

**Table 4: Results for the bulk viscosity of body wash formulations F10 to F16**

| **Formulation** | **Thickener** | **Bulk viscosity (cps)** |
|---|---|---|
| **F10 (CE)** | **N1 (CE)** | 500 |
| **F11 (CE)** | **Carbopol** ^{®}**940 (CE)** | 2 000 |
| **F12 (CE)** | **Lingel 940 (CE)** | 1 700 |
| **F13 (Inv)** | **N7 (Inv)** | 2 400 |
| **F14 (Inv)** | **N3 (Inv)** | 1 800 |
| **F15 (Inv)** | **N28 (Inv)** | 1 500 |
| **F16 (Inv)** | **N9 (Inv)** | 1 600 |

### Example 5: After sun gel formulations

Different after sun gel formulation F17 to F21 were produced by replacing the nature of the thickener. The nature of the different thickeners comprised in formulations F17 to F21 is listed in table 5.

### Ingredients (by weight %):

- Deionized water: *q.s.*
- Glycerin: 3.0
- Thickener: 0.4
- Panthenol: 2.0
- Aloe vera: 5.0
- Phenoxyethanol: 0.2.

### After sun gel formulation protocol:

The thickener was slowly incorporated in deionized water while stirring at 200 rpm, until a lumps-free solution was obtained. For formulations F17 and F18 comprising the synthetic thickeners, triethanolamine was slowly added to the mixture for 30 minutes, to adjust the pH to a range of 5.5 to 6.5.

All the other ingredients were added while maintaining agitation at 800 rpm. The pH was adjusted, if needed, by using triethanolamine or citric acid to keep it in the range of 5.5 to 6.5. The mixture was then mixed until it formed a smooth and homogeneous formulation.

The bulk viscosity of the different formulations F17 to F21 was measured and the results are summarized in Table 5.

**Table 5: Bulk viscosity of the after-sun gel formulations F17 to F21**

| **Formulation** | **Thickener** | **Bulk viscosity at t=1month (cps)** | **Bulk viscosity at t=12 months (cps)** |
|---|---|---|---|
| **F17 (CE)** | **Acritamer 940 (CE)** | 43 000 | >50 000 |
| **F18 (CE)** | **Aquastyle 940 (CE)** | 47 000 | >50 000 |
| **F19 (Inv)** | **N23 (Inv)** | 41 000 | 44 000 |
| **F20 (CE)** | **N29 (CE)** | 15 000 | 16 700 |
| **F21 (CE)** | **N30 (CE)** | 25 000 | 24 400 |

### Example 6: Baby care & cleansing gel formulations

Different baby care & cleansing gel formulations F22 and F23 were produced by replacing the nature of the thickener. The nature of the different thickeners comprised in formulations F22 and F23 is resumed in table 6.

### Ingredients (by weight %):

- Deionized water: *q.s.*
- Decyl glucoside: 15.0
- Glycerin: 5.0
- Thickener: 0.3
- Chamomile extract: 5.0
- Phenoxyethanol: 0.2

### Baby care & cleansing gel formulation protocol:

The thickener was slowly incorporated in deionized water while stirring at 200 rpm, until a lumps-free solution was obtained. For formulation F22 comprising the synthetic thickener, triethanolamine was slowly added to the mixture for 30 minutes, to adjust the pH to a range of 5.5 to 6.5.

All the other ingredients were added while maintaining agitation at 200 rpm. The pH was adjusted, if needed, by using triethanolamine or citric acid to keep it in the range of 5.5 to 6.5. The mixture was then mixed until it formed a smooth and homogeneous formulation.

The bulk viscosity of the different formulations F22 and F23 was measured, and the results are listed in Table 6.

**Table 6: Bulk viscosity of baby care & cleansing gel formulations F22 and F23**

| **Formulation** | **Thickener** | **Bulk viscosity (cps)** | **Clarity (% transmittance)** |
|---|---|---|---|
| **F22 (CE)** | **Carbopol**^{®} **940 (CE)** | 24 000 | 82 |
| **F23 (Inv)** | **N28 (Inv)** | 22 400 | 91 |

Formulation F23 according to the invention is more transparent than formulation F22. This feature is particularly advantageous when a transparent aspect is desired for some personal care formulations.

### Example 7: Face care moisturizing gel formulations

Different face care moisturizing gel formulations F24 and F25 were produced by replacing the nature of the thickener. The nature of the different thickeners comprised in formulations F24 and F25 is resumed in table 7.

### Ingredients (by weight %):

- Deionized water: *q.s.*
- Glycerin: 4.8
- Thickener: 0.5
- Niacinamide: 2.0
- Aloe vera: 10
- Phenoxyethanol: 0.2.

### Face care moisturizing gel formulation protocol:

The thickener was slowly incorporated in deionized water while stirring at 200 rpm, until a lumps-free solution was obtained. For formulation F24 comprising the synthetic thickener, triethanolamine was slowly added to the mixture for 30 minutes, to adjust the pH to a range of 5.5 to 6.5.

All the other ingredients were added while maintaining agitation at 200 rpm. The pH was adjusted, if needed, by using triethanolamine or citric acid to keep it in the range of 5.5 to 6.5. The mixture was then mixed until it formed a smooth and homogeneous formulation.

The bulk viscosity of the different formulations F24 and F25 was measured, and the results are listed in Table 7.

**Table 7: Bulk viscosity of face care moisturizing gel formulations F24 and F25**

| **Formulation** | **Thickener** | **Bulk viscosity (cps)** |
|---|---|---|
| **F24 (CE)** | **Rheocare^{®} C plus (CE)** | 33 800 |
| **F25 (Inv)** | **N20 (Inv)** | 37 200 |

### Example 8: Face hair styling gel formulations

Different face hair styling gel formulations F26 and F27 were formulated by replacing the nature of the thickener. The nature of the different thickeners comprised in formulations F26 and F27 is summarized in table 8.

### Ingredients (by weight %):

- Deionized water: *q.s.*
- Glycerin: 3.0
- Thickener: 0.6
- Polyquaternium 4: 5.0
- Phenoxyethanol: 0.2.

### Face hair styling gel formulation protocol:

The thickener was slowly incorporated in deionized water while stirring at 200 rpm, until a lumps-free solution was obtained. For formulation F26 comprising the synthetic thickener, triethanolamine was slowly added to the mixture for 30 minutes, to adjust the pH to a range of 5.5 to 6.5.

All the other ingredients were added while maintaining agitation at 1200 rpm. The pH was adjusted, if needed, by using triethanolamine or citric acid to keep it in the range of 5.5 to 6.5. The mixture was then mixed until it formed a smooth and homogeneous formulation.

The bulk viscosity of the different formulations F26 and F27 was measured, and the results are summarized in Table 8.

**Table 8: Bulk viscosity of face hair styling gel formulations F26 and F27**

| **Formulation** | **Thickener** | **Bulk viscosity (cps)** |
|---|---|---|
| **F26 (CE)** | **TC-Carbomer^{®} 640 (CE)** | 64 400 |
| **F27 (Inv)** | **N22 (Inv)** | 67 200 |

### Example 9: Soothing facial gel formulations with sodium chloride

Different soothing facial gel formulations F28 and F29 were produced by replacing the nature of the thickener. The nature of the different thickeners comprised in formulations F28 and F29 is summarized in table 9.

### Ingredients (by weight %):

- Deionized water: *q.s.*
- Glycerin: 4.0
- Thickener: 0.4
- Sodium chloride: 0.6
- Aloe vera extract: 6.0
- Panthenol: 2.0
- Phenoxyethanol: 0.8
- Fragrance: 0.3.

### Soothing facial gel formulation protocol:

The thickener was dispersed in deionized water, comprising the sodium chloride, by using a low-speed mixer at 200 rpm, until a lumps-free solution was obtained. Glycerin was then added, and the solution was mixed for 20 minutes. For formulation F28 comprising the synthetic thickener, triethanolamine was slowly added to the mixture for 30 minutes, to adjust the pH to a range of 5.5 to 6.5.

The mixture formed was then stirred until it became clear. Aloe vera extract, panthenol glycol, phenoxyethanol, fragrance, were finally introduced. The formulation was then mixed until it was smooth and homogeneous. The pH was adjusted, if needed, by using triethanolamine or citric acid to keep it in the range of 5.5 to 6.5.

The bulk viscosity of the different formulations F28 and F29 were measured, and the results are summarized in Table 9.

**Table 9: Results for the bulk viscosity of the soothing facial gel formulations F28 and F29**

| **Formulation** | **Thickener** | **Bulk viscosity (cps)** |
|---|---|---|
| **F28 (CE)** | **Carbopol^{®} 940 (CE)** | 8 100 |
| **F29 (Inv)** | **N22 (Inv)** | 11 700 |

### Example 10: High-salt personal care gel

Different high-salt personal care gel formulations F30 to F32 were formulated by replacing the nature of the thickener. The nature of the different thickeners comprised in formulations F30 to F32 is summarized in table 10.

### Ingredients (by weight %):

- Deionized Water: *q.s.*
- Sodium Chloride: 15
- Thickener: 0.5
- Glycerin: 3.0
- Phenoxyethanol: 0.5
- Fragrance: 0.2
- Colorant: 1.8.

### Personal care gel formulation protocol:

The thickener was dispersed in deionized water, comprising the sodium chloride, by using a low-speed mixer at 200 rpm, until a lumps-free solution was obtained. For formulation F30 comprising the synthetic thickener, triethanolamine was slowly added to the mixture in 30 minutes, to adjust the pH to a range of 5.5 to 6.5.

All the other ingredients were added while maintaining agitation. The pH was adjusted, if needed, by using triethanolamine or citric acid to keep it in the range of 5.5 to 6.5. The mixture was then mixed until it formed a smooth and homogeneous formulation.

The bulk viscosity of the different formulations F30 to F32 was measured, and the results are summarized in Table 10.

**Table 10: Bulk viscosity of soothing facial gel formulations F30 to F32**

| **Formulation** | **Thickener** | **Bulk viscosity (cps)** |
|---|---|---|
| **F30 (CE)** | **Carbopol 940**^{®} **(CE)** | 3 200 |
| **F31 (Inv)** | **N22 (Inv)** | 5 700 |
| **F32 (Inv)** | **N8 (Inv)** | 5 600 |

As demonstrated in all the examples, the natural thickener according to the invention offers to the personal care composition comprising a wide range of viscosity depending on the ratio between the crosslinker and the natural polymer in non-hydrated form. In contrast, the natural thickeners obtained by a process that does not involve a crosslinker have only low bulk viscosity values. These ranges of viscosity are comparable with those obtained with synthetic thickeners. Additionally, the natural thickeners according to the invention show better biodegradability than the synthetic thickeners.

## Claims

1. Personal care composition comprising from 0.1% to 10% by weight of a natural thickener, relative to the weight of the personal care composition, wherein the natural thickener has a particle size of between 1 µm and 1 000 µm and is prepared according to at least the following steps:
i) preparing a mixture M1 comprising at least one crosslinker and a solvent S1 able to solubilize, to disperse or to suspend the at least one crosslinker,
wherein a weight ratio between the at least one crosslinker and the solvent S1 in the mixture M1 is between 0.1/99.9 and 30/70;
ii) forming the natural thickener by mixing the mixture M1 with at least one natural polymer in non-hydrated particle form, wherein a weight ratio between the at least one crosslinker and the at least one natural polymer in non-hydrated particle form is between 0.01/99.9 and 10/90, and wherein the mixing lasts between 1 minute and 150 minutes, at a temperature comprised between 20°C and 160°C.

2. Personal care composition according to claim 1, ***wherein*** the crosslinker is selected from the group consisting of: polyepoxys; polycarboxylic acids; anhydride derivatives; polycarbamides; polyamides; polyamines; polyaldehydes; polyols; polyvalent inorganic salts; and mixtures thereof.

3. Personal care composition according to claim 1 or 2, ***wherein*** the solvent S1 is selected from the group consisting of: C₁-C₄ alcohols; C₃-C₄ ketones; mineral oils; vegetable oils; C₁-C₈ linear ethers; C₁-C₈ cyclic ethers; C₁-C₈ branched ethers; and mixtures thereof.

4. Personal care composition according to any one of the preceding claims, ***wherein*** the solvent S1 is C₁-C₄ alcohol selected from the group consisting of: methanol; ethanol; 1-propanol; isopropanol; 2-methyl-1-propanol; tert-butanol; 1-butanol; 2-butanol; and mixtures thereof.

5. Personal care composition according to any one of the preceding claims, ***wherein*** the natural polymer in non-hydrated particle form is not crosslinked before the mixing with mixture M1.

6. Personal care composition according to any one of the preceding claims, ***wherein*** the natural polymer in non-hydrated particle form is a polysaccharide.

7. Personal care composition according to any one of the preceding claims, ***wherein*** mixture M1 is sprayed on the natural polymer in non-hydrated particle form during the mixing step ii).

8. Personal care composition according to any one of the preceding claims, ***wherein*** the natural polymer in non-hydrated particle form is grinded and sieved to a particle size of between 1 µm and 1 000 µm before step ii).

9. Personal care composition according to any one of the preceding claims, ***wherein*** the natural thickener is grinded and sieved to a particle size of between 1 µm and 1 000 µm in step ii).

10. Personal care composition according to any one of the preceding claims, ***wherein*** the personal care composition comprises one or more active ingredient.

11. Personal care composition according to claim 10, ***wherein*** the personal care composition comprises between 0.1 and 30% by weight of active ingredient, relative to the weight of the personal care composition.

12. Personal care composition according to any one of the preceding claims, ***wherein*** the personal care composition comprises one or more additives and/or one or more excipients.

13. Personal care composition according to claim 11 or 12, ***wherein*** the personal care composition comprises, relative to the weight of the personal care composition:
- between 0.1% and 10% of one or more natural thickener(s);
- between 0.1% and 30% of one or more active ingredient(s);
- between 0.1% and 30% of one or more additive(s) and/or excipient(s);
- between 30% and 99.7% of a continuous phase selected from water, C₁-C₄ alcohol(s), oil(s), wax(es) and mixtures thereof.

14. Process for preparing a natural thickener comprising the following steps:
i) preparing a mixture M1 comprising at least one crosslinker and a solvent S1 able to solubilize, to disperse or to suspend the at least one crosslinker,
wherein a weight ratio between the at least one crosslinker and solvent S1 in the mixture M1 is between 0.1/99.9 and 30/70;
ii) forming the natural thickener by mixing the mixture M1 with at least one natural polymer in non-hydrated particle form, wherein a weight ratio between the at least one crosslinker and the at least one natural polymer in non-hydrated particle form is between 0.01/99.9 and 10/90, and wherein the mixing lasts between 1 minute and 150 minutes, at a temperature comprised between 20°C and 160°C.

15. Process according to claim 14, ***wherein*** said process is carried out using energy derived from a heat pump, a waste heat network, a renewable source, a fuel cell, a lithium battery, or a nuclear power plant.

16. Natural thickener prepared by the process according to claim 14 or 15.
